# EUROPÄISCHE PATENTSCHRIFT

(11) **EP 2 125 759 B1**
(45) Veröffentlichungstag und Bekanntmachung des Hinweises auf die Patenterteilung: **03.08.2011**
(21) Anmeldenummer: 08708139.4
(22) Anmeldetag: 24.01.2008
(51) Int. Cl.: C07D 265/36

(54) **VERFAHREN ZUR HERSTELLUNG VON BETAMIMETIKA**
METHOD FOR PRODUCING BETAMIMETICS
PROCÉDÉ POUR PRODUIRE DES BÊTAMIMÉTIQUES

(30) Priorität: 25.01.2007 EP 07101130
(43) Veröffentlichungstag der Anmeldung: 02.12.2009
(73) Patentinhaber: Boehringer Ingelheim International GmbH, 55216 Ingelheim am Rhein (DE)
(72) Erfinder: RODRIGUEZ DEHLI, Juan M., 55216 Ingelheim am Rhein (DE); HAGENKOETTER, Robert, 55216 Ingelheim am Rhein (DE); SCHUL, Michael, 55216 Ingelheim am Rhein (DE); STANGE, Christian, 55216 Ingelheim am Rhein (DE)
(74) Vertreter: Hammann, Heinz
(86) Internationale Anmeldenummer: PCT/EP2008/050800
(87) Internationale Veröffentlichungsnummer: WO 2008/090193

(56) Entgegenhaltungen:
- WO-A-2005/111005

## Beschreibung

Die vorliegende Erfindung betrifft ein Verfahren zur Herstellung von Betamimetika der Formel **1**, 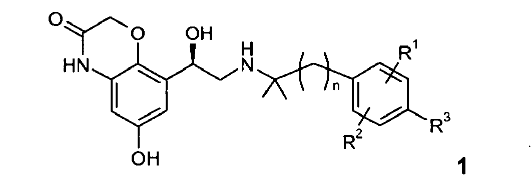 worin
- n: 1 oder 2;
- R¹: Wasserstoff, Halogen, C₁₋₄-Alkyl oder O-C₁₋₄-Alkyl;
- R²: Wasserstoff, Halogen, C₁₋₄-Alkyl oder O-C₁₋₄-Alkyl;
- R³: Wasserstoff, C₁₋₄-Alkyl, OH, Halogen, O-C₁₋₄-Alkyl, O-C₁₋₄-Alkylen-COOH, O-C₁₋₄-Alkylen-COO-C₁₋₄-Alkyl;
bedeuten.

### HINTERGRUND DER ERFINDUNG

Betamimetika (ß-adrenerge Substanzen) sind aus dem Stand der Technik bekannt. Beispielsweise sei diesbezüglich auf die Offenbarung der US 4,460,581 verwiesen, die Betamimetika zur Therapie unterschiedlichster Erkrankungen vorschlägt.

WO2005/111005 wird als nächster Stand der Technik, betrachtet.

Es ist daher Aufgabe der vorliegenden Erfindung ein Verfahren zur Herstellung von Betamimetika bereitzustellen,

### DETAILLIERTE BESCHREIBUNG DER ERFINDUNG

Die vorliegende Erfindung betrifft ein Verfahren zur Herstellung einer Verbindung der der Formel **1**, 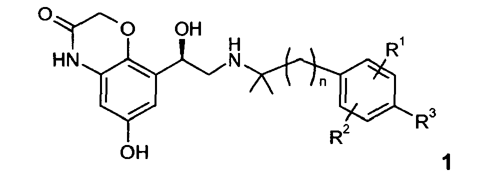 worin
- n: 1 oder 2;
- R¹: Wasserstoff, Halogen, C₁₋₄-Alkyl oder O-C₁₋₄-Alkyl;
- R²: Wasserstoff, Halogen, C₁₋₄-Alkyl oder O-C₁₋₄-Alkyl;
- R³: Wasserstoff, C₁₋₄-Alkyl, OH, Halogen, O-C₁₋₄-Alkyl, O-C₁₋₄-Alkylen-COOH, O-C₁₋₄-Alkylen-COO-C₁₋₄-Alkyl;
bedeuten, dadurch gekennzeichnet, dass eine Verbindung der Formel **1a**, 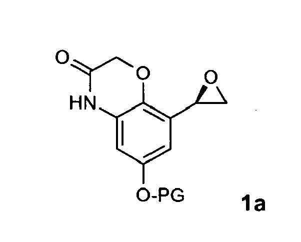 worin PG für eine Schutzgruppe steht, mit einer Verbindung der Formel **1b**, 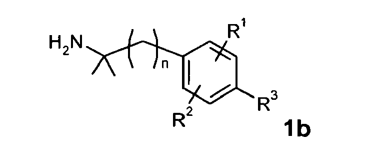 worin R¹, R², R³ und n die oben genannte Bedeutung haben, in einem organischen Lösungsmittel zu einer Verbindung der Formel **1c**, 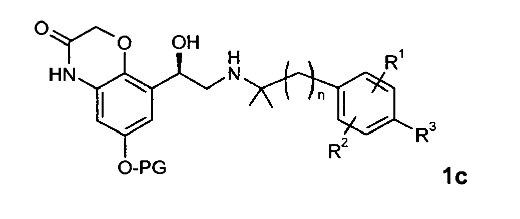 worin R¹, R², R³, n und PG die oben genannten Bedeutungen haben, umgesetzt und aus dieser durch Abspaltung der Schutzgruppe PG die Verbindung der Formel **1** erhalten wird.

Bevorzugt ist das obige Verfahren zur Herstellung von Verbindungen der Formel 1, worin
- n: 1 oder 2;
- R¹: Wasserstoff, Halogen oder C₁₋₄-Alkyl;
- R²: Wasserstoff, Halogen oder C₁₋₄-Alkyl;
- R³: Wasserstoff, C₁₋₄-Alkyl, OH, Halogen, O-C₁₋₄-Alkyl, O-C₁₋₄-Alkylen-COOH oder O-C₁₋₄-Alkylen-COO-C₁₋₄-Alkyl;
bedeuten.

Bevorzugt ist das obige Verfahren zur Herstellung von Verbindungen der Formel **1**, worin
- n: 1 oder 2;
- R¹: Wasserstoff, Fluor, Chlor, Methyl oder Ethyl;
- R²: Wasserstoff, Fluor, Chlor, Methyl oder Ethyl;
- R³: Wasserstoff, C₁₋₄-Alkyl, OH, Fluor, Chlor, Brom, O-C₁₋₄-Alkyl, O-C₁₋₄-Alkylen-COOH, O-C₁₋₄-Alkylen-COO-C₁₋₄-Alkyl;
bedeuten.

Bevorzugt ist das obige Verfahren zur Herstellung von Verbindungen der Formel **1**, worin
- n: 1 oder 2;
- R¹: Wasserstoff, Methyl oder Ethyl;
- R²: Wasserstoff, Methyl oder Ethyl;
- R³: Wasserstoff, Methyl, Ethyl, OH, Methoxy, Ethoxy, O-CH₂-COOH, O-CH₂-COO-Methyl oder O-CH₂-COO-Ethyl;
bedeuten.

Bevorzugt ist das obige Verfahren zur Herstellung von Verbindungen der Formel **1**, worin
- n: 1 oder 2;
- R¹: Wasserstoff oder Methyl;
- R²: Wasserstoff oder Methyl;
- R³: Wasserstoff, Methyl, OH, Methoxy, O-CH₂-COOH oder O-CH₂-COO-Ethyl;
bedeuten.

Im erfindungsgemäßen Verfahren wird eine Verbindung der Formel **1a** in Toluol mit einer Verbindung der Formel **1b** umgesetzt. Die Verbindung der Formel **1b** kann dabei als Base vorliegen. Dafür wird das entsprechende Salz (z.B. Hydrochlorid) mit einem Überschuss einer starken Base (Natronlauge, Kalilauge, usw.) in ein organisches Lösungsmittel extrahiert, bevorzugt Toluol.

Bezogen auf eingesetzte Verbindung **1a** gelangen erfindungsgemäß bevorzugt wenigstens stöchiometrische Mengen an Verbindung **1b** zum Einsatz. Gegebenenfalls kann Verbindung 1 b auch im Überschuss, beispielsweise in bis zu 3 Äquivalenten, vorzugsweise in bis zu 2,5 Äquivalenten, besonders bevorzugt in etwa 1 bis 2, gegebenenfalls in 1 bis 1.5 Äquivalenten bezogen auf eingesetzte Verbindung **1a** eingesetzt werden.

Die Umsetzung erfolgt vorzugsweise bei erhöhter Temperatur, vorzugsweise bei einer Temperatur von über 40°C, besonders bevorzugt bei einer Temperatur von über 50°C. Besonders bevorzugt wird Reaktionsmischung bis zur Siedetemperatur des eingesetzten Lösungsmittels erhitzt.

Bei dieser Temperatur wird die Umsetzung sodann in einem Zeitraum von 3 bis 9 Stunden, bevorzugt 4-7, vorzugsweise etwa 6 Stunden durchgeführt.

Nach vollendeter Reaktion wird Methylacetat zugegeben und die resultierende Lösung filtriert. Das Filtrat wird auf 50°C erwärmt und angesäuert, bevorzugt mit einer Mineralsäure, besonders bevorzugt mit Salzsäure und nach einer Dauer von etwa 10 Minuten bis 12 Stunden, vorzugsweise 20 Minuten bis 6 Stunden, besonders bevorzugt 30 Minuten bis 3 Stunden wird das Produkt abfiltriert. Bei der Zugabe von der Säure kann mit Kristallen der Verbindung **1c** angeimpft werden, beispielsweise nach Zugabe von etwa 5% der zum Einsatz gelangenden Säure.

Die Abspaltung der Schutzgruppe PG aus Verbindungen der Formel **1c** erfolgt bevorzugt mittels Hydrierung in einem geeigneten Lösungsmittel. Als geeignete Lösungsmittel kommen organische Lösungsmittel, bevorzugt organische, polare Lösungsmittel in Betracht, besonders bevorzugte Lösungsmittel sind ausgewählt aus der Gruppe bestehend aus Tetrahydrofuran, verschiedene C₃₋₈-Ester und C₁₋₈-Alkohole. Erfindungsgemäß bevorzugt gelangen als Lösungsmittel Tetrahydrofuran, Ethanol und Methanol zum Einsatz, wobei Ethanol und Methanol besondere Bedeutung zukommt. Zur Hydrierung im erfindungsgemäßen Verfahren werden bevorzugt Katalysatoren in Gegenwart von Wasserstoff eingesetzt. Bevorzugte Katalysatoren sind geeignete Übergangsmetallkatalysatoren, bevorzugt heterogene Übergangsmetallkatalysatoren, besonders bevorzugt Palladium-haltige Katalysatoren, insbesondere ein Palladium-Kohle-Gemisch.

Die Hydrierung erfolgt bevorzugt in Gegenwart eines Überschuss von Wasserstoff. Dieser wird erfindungsgemäß durch einen Wasserstoffdruck von 1 bar bis 10 bar, bevorzugt zwischen 2 und 7 bar, besonders bevorzugt zwischen 2,5 und 4,5 bar, bereitgestellt.

Bevorzugt wird die Hydrierung bei 60° C durchgeführt. Nach vollendeter Reaktion wird der Katalysator, bevorzugt durch Filtration, entfernt.

Danach wird die Lösung mit Isopropylalkohol (IPA) verdünnt und das Produkt umkristallisiert. Bevorzugt wird dabei die Lösung teilweise eingeengt und das Produkt durch Abkühlen der Lösung auskristallisiert.

Erfindungsgemäß bevorzugt ist ein Verfahren, worin die Verbindung der Formel **1a** durch Umsetzung einer Verbindung der Formel **2a**, 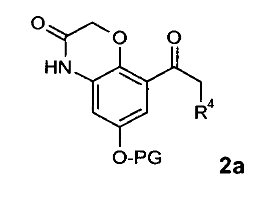 worin PG die in Anspruch 1 genannte Bedeutung hat und R⁴ Halogen, bevorzugt Brom oder Chlor, bedeutet, hergestellt wird.

Im erfindungsgemäßen Verfahren wird eine Verbindung der Formel **2a** in einem geeigneten Lösungsmittel mit (-)-DIP-Chlorid (Diisopinocampheylchloroboran) umgesetzt. Als geeignete Lösungsmittel kommen bevorzugt organische Lösungsmittel in Betracht. Bevorzugte Lösungsmittel sind ausgewählt aus der Gruppe bestehend aus Diethylether, *tert*-Butyl-methylether 2-Methyltetrahydrofuran, Tetrahydrofuran, Toluol und Dioxan. Erfindungsgemäß besonders bevorzugt gelangen als Lösungsmittel zum Einsatz *tert-*Butyl-methylether, Tetrahydrofuran und Dioxan, wobei Dioxan und Tetrahydrofuran besondere Bedeutung zukommt.

Das (-)-DIP-Chlorid kann in Reinform oder in Form einer Lösung, bevorzugt in einem inerten, organischen Lösungsmittel, besonders bevorzugt einem aliphatischen Lösungsmittel, insbesondere Pentan, Hexan, Heptan oder Octan, insbesondere Heptan eingesetzt werden.

Die Zugabe des (-)-DIP-Chlorids erfolgt bei verminderter Temperatur im Reaktionsmedium, bevorzugt liegt die Temperatur dabei unter 0°C, besonders bevorzugt unter -10°C, insbesondere erfolgt die Zugabe bei -20 bis -40°C.

Die Zugabe des (-)-DIP-Chlorids erfolgt über einen Zeitraum von 10 min bis 6 Stunden, bevorzugt von 30 min bis 4 Stunden, besonders bevorzugt von 1 bis 3 Stunden. Insbesondere erfolgt die Zugabe in einem Zeitraum von 70 bis 110 min.

Bezogen auf eingesetzte Verbindung **2a** gelangen erfindungsgemäß bevorzugt wenigstens stöchiometrische Mengen an (-)-DIP-Chlorid zum Einsatz. Gegebenenfalls kann das (-)-DIP-Chlorid auch im Überschuss, beispielsweise in bis zu 3 Äquivalenten, vorzugsweise bis zu 2,5 Äquivalente, besonders bevorzugt in etwa 1,5 bis 2,5 Äquivalenten, insbesondere in etwa 1,8 Äquivalenten bezogen auf eingesetzte Verbindung **2a** eingesetzt werden.

Nach erfolgter Zugabe des (-)-DIP-Chlorids wird das Reaktionsgemisch über einen Zeitraum von 10 min bis 4 Stunden, bevorzugt von 30 min bis 3 Stunden, besonders bevorzugt von 40 bis 80 min, insbesondere wird das Reaktionsgemisch nach erfolgter Zugabe noch für Zeitraum von 50 bis 70 min gerührt. Während diesem Zeitraum wird das Reaktionsgemisch auf eine Temperatur unter 0°C, besonders bevorzugt unter -10°C, insbesondere bei -20 bis -40°C, eingestellt.

Danach wird, bezogen auf die eingesetzte Menge an (-)-DIP-Chlorid, eine wenigstens stöchiometrische Menge an Natriumhydroxid (NaOH), in Wasser gelöst, zugegeben. Gegebenenfalls kann das NaOH auch im Überschuss, beispielsweise in bis zu 3 Äquivalenten, vorzugsweise in bis zu 2,5 Äquivalenten, besonders bevorzugt in etwa 1,5 bis 2,5 Äquivalenten, bezogen auf die eingesetzte Menge DIP-Chlorid, eingesetzt werden. Bevorzugt wird in der Reaktionsmischung nach erfolgter Zugabe von NaOH ein pH-Wert von 12 bis 14, besonders bevorzugt von 12,5 bis 13,5, insbesondere von 12,7 bis 13,3, gemessen.

Nachdem der gewünschte pH-Wert eingestellt wurde, wird das Reaktionsgemisch über einen Zeitraum von 10 min bis 4 Stunden, bevorzugt von 30 min bis 3 Stunden, besonders bevorzugt von 40-80 min, insbesondere wird das Reaktionsgemisch noch für Zeitraum von 50-70 min gerührt. Während diesem Zeitraum wird das Reaktionsgemisch auf eine Temperatur von 0 bis 40°C, besonders bevorzugt von 10 bis 30°C, insbesondere von 15 bis 25°C, eingestellt. Anschließend wird das Reaktionsgemisch mit einer Säure, bevorzugt einer Mineralsäure, besonders bevorzugt mit Salzsäure, auf einen pH-Wert von 7 bis 10, besonders bevorzugt von 8 bis 9, insbesondere von 8,2 bis 8,8, eingestellt.

Abschließend kann das Produkt durch Extraktion mit einem organischen Lösungsmittel (bevorzugt Essigester) aus dem Reaktionsgemisch isoliert werden und durch Fällung mit einem weiteren geeigneten organischen Lösungsmittel (bevorzugt Methyl-tert-butylether) als Feststoff erhalten werden.
Vor der Fällung kann das Lösungsmittelgemisch teilweise abdestilliert werden.

Erfindungsgemäß bevorzugt ist ein Verfahren, worin die Verbindung der Formel **1b** durch Umsetzung einer Verbindung der Formel **2b**, 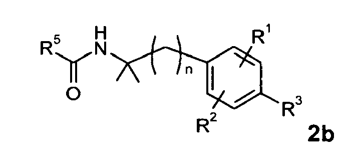 worin R¹, R², R³ und n die in den Ansprüchen 1 bis 5 genannte Bedeutung haben und
- R⁵: C₁₋₆-alkyl, bevorzugt Me;
bedeutet, hergestellt wird.

Im erfindungsgemäßen Verfahren wird eine Verbindung der Formel **2b** in einem geeigneten Lösungsmittel mit Natriumhydroxid umgesetzt. Als geeignete Lösungsmittel kommen organische Lösungsmittel in Betracht, besonders bevorzugte Lösungsmittel sind ausgewählt aus der Gruppe bestehend aus Ethanol, 2-Ethoxyethanol, Ethylenglykol, Butoxyethanol, Methoxypropanol, Propylenglykol oder Gemische davon. Erfindungsgemäß besonders bevorzugt gelangen 2-Ethoxyethanol oder Ethylenglykol oder ein Gemisch davon als Lösungsmittel zum Einsatz. Bevorzugt besteht das Gemisch aus 2-Ethoxyethanol und Ethylenglykol (2:1).

Bezogen auf eingesetzte Verbindung **2b** gelangen erfindungsgemäß bevorzugt wenigstens stöchiometrische Mengen der starken Base zum Einsatz. Gegebenenfalls kann die starke Base auch im Überschuss, beispielsweise in bis zu 8 Äquivalenten, vorzugsweise in bis zu 6 Äquivalenten, bevorzugt in etwa 2 bis 6, besonders bevorzugt in 4,5 bis 5,5 Äquivalenten, bezogen auf eingesetzte Verbindung **2b,** eingesetzt werden.

Die Umsetzung erfolgt vorzugsweise bei erhöhter Temperatur, vorzugsweise bei einer Temperatur von über 100°C, besonders bevorzugt unter Rückfluss des Lösungsmittels und während 2-3 Stunden.

Anschließend wird das Reaktionsgemisch zur Extraktion mit einem Lösungsmittel und Wasser verdünnt. Als Lösungsmittel kommt Toluol, Xylol, Heptan, Methylcyclohexan oder *tert*-Butyl-methylether, bevorzugt Toluol oder Xylol, besondere Bedeutung zu. Die wässrige Phase wird entfernt, die organische Phase in weiteren Reinigungsschritten mit Wasser extrahiert. Das Wasser kann dabei durch gängige Zusatzstoffe sauer, neutral oder alkalisch sein. Bevorzugt wird die organische Phase mit basischem oder neutralem Wasser extrahiert. Das Produkt wird aus der organischen Phase unter Zugabe einer geeigneten Menge HCl bei mindestens 60°C, bevorzugt ca. 80°C, anschließender Azeotropdestillation und abschließendem Abkühlen bis Raumtemperatur als HCl-Salz isoliert.

### VERWENDETE BEGRIFFE UND DEFINTIONEN

Unter einem "organischen Lösungsmittel" wird im Rahmen der Erfindung ein organischer, niedermolekularer Stoff verstanden, der andere organische Stoffe auf physikalischem Wege zur Lösung bringen kann. Voraussetzung für die Eignung als Lösungsmittel ist, dass sich beim Lösungsvorgang weder der lösende noch der gelöste Stoff chemisch verändern, dass also die Komponenten der Lösung durch physikalische Trennverfahren wie Destillation, Kristallisation, Sublimation, Verdunstung, Adsorption in der Originalgestalt wieder gewonnen werden können. Aus verschiedenen Gründen können nicht nur die reinen Lösungsmittel, sondern Gemische, die die Lösungseigenschaften vereinigen, verwendet werden. Beispielsweise seien genannt:
- Alkohole, bevorzugt Methanol, Ethanol, Propanol, Butanol, Octanol, Cyclohexanol;
- Glykole, bevorzugt Ethylenglykol, Diethylenglykol;
- Ether / Glykolether, bevorzugt Diethylether, fert-Butyl-methylether, Dibutylether, Anisol, Dioxan, Tetrahydrofuran, Mono-, Di-, Tri-, Polyethylenglykolether;
- Ketone, bevorzugt Aceton, Butanon, Cyclohexanon;
- Ester, bevorzugt Essigsäureester, Glykolester;
- Amide u.a. Stickstoff-Verbindungen, bevorzugt Dimethylformamid, Pyridin, *N-*Methylpyrrolidon, Acetonitril;
- Schwefel-Verbindungen, bevorzugt Schwefelkohlenstoff, Dimethylsulfoxid, Sulfolan;
- Nitro-Verbindungen, bevorzugt Nitrobenzol;
- Halogenkohlenwasserstoffe, bevorzugt Dichlormethan, Chloroform, Tetrachlormethan, Tri- und Tetrachlorethen, 1,2-Dichlorethan, Chlorfluorkohlenstoffe;
- aliphatische oder alicyclische Kohlenwasserstoffe, bevorzugt Benzine, Petrolether, Cyclohexan, Methylcyclohexan, Decalin, Terpen-L.; oder
- aromatische Kohlenwasserstoffe, bevorzugt Benzol, Toluol, *o*-Xylol, *m*-Xylol, *p*-Xylol; oder entsprechende Gemische davon.

Unter dem Begriff "C₁₋₄-Alkyl" (auch soweit sie Bestandteil anderer Reste sind) werden verzweigte und unverzweigte Alkylgruppen mit 1 bis 4 Kohlenstoffatomen verstanden. Beispielsweise werden hierfür genannt: Methyl, Ethyl, *n*-Propyl, *iso*-Propyl, *n*-Butyl, *iso-*Butyl, sec-Butyl oder *tert-*Butyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₄-Alkylen" (auch soweit sie Bestandteil anderer Reste sind) werden verzweigte und unverzweigte Alkylengruppen mit 1 bis 4 Kohlenstoffatomen verstanden. Beispielsweise werden hierfür genannt: Methylen, Ethylen, Propylen, 1-Methylethylen, Butylen, 1-Methylpropylen, 1,1-Dimethylethylen oder 1,2-Dimethylethylen. Sofern nicht anders beschrieben, umfassen die Definitionen Propylen und Butylen alle denkbaren isomeren Formen der jeweiligen Reste gleicher Kohlenstoffanzahl. So umfasst beispielsweise Propylen auch 1-Methylethylen und Butylen umfasst 1-Methylpropylen, 1,1-Dimethylethylen, 1,2-Dimethylethylen.

Unter dem Begriff "C₁₋₈-Alkohol" werden verzweigte und unverzweigte Alkohole mit 1 bis 8 Kohlenstoffatomen verstanden und einer oder zwei Hydroxygruppen. Bevorzugt sind Alkohole mit 1 bis 4 Kohlenstoffatome. Beispielsweise werden hierfür genannt: Methanol, Ethanol, *n*-Propanol, *iso*-Propanol, *n*-Butanol, *iso*-Butanol, *sec*-Butanol oder *tert*-Butanol. Gegebenenfalls werden für vorstehend genannte Moleküle auch die Abkürzungen MeOH, EtOH, *n*-PrOH, *i*-PrOH, *n*-BuOH, *i*-BuOH, *t*-BuOH, etc. verwendet. Sofern nicht anders beschrieben, umfassen die Definitionen Propanol, Butanol, Pentanol und Hexanol alle denkbaren isomeren Formen der jeweiligen Reste. So umfasst beispielsweise Propanol *n-*Propanol und *iso*-Propanol, Butanol umfasst *iso*-Butanol, *sec*-Butanol und *tert*-Butanol etc.

Unter dem Begriff "C₃₋₈-Ester" werden verzweigte und unverzweigte Ester mit insgesamt 3 bis 8 Kohlenstoffatomen verstanden. Bevorzugt sind Ester der Essigsäure mit 3 bis 6 Kohlenstoffatome. Beispielsweise werden hierfür genannt: Methylacetat, Ethylacetat, *n*-Propylacetat, *i*-Propylacetat oder n-Butylacetat, bevorzugt ist Ethylacetat.

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

"Schutzgruppen" in Sinne der vorliegenden Erfindung sind als Sammelbezeichnung für solche organische Reste zu verstehen, mit denen bestimmte funktionelle Gruppen eines mehrere aktive Zentren enthaltenden Moleküls vorübergehend gegen den Angriff von Reagenzien geschützt werden können, so dass Reaktionen nur an den gewünschten (ungeschützten) Stellen stattfinden. Die Schutzgruppen sollen unter milden Bedingungen selektiv einzuführen sein. Sie müssen für die Dauer des Schutzes unter allen Bedingungen der durchzuführenden Reaktionen und Reinigungsoperationen stabil sein; Racemisierungen und Epimerisierungen müssen unterdrückt werden. Schutzgruppen sollen wieder unter milden Bedingungen selektiv und idealerweise mit hoher Ausbeute abspaltbar sein. Die Wahl einer geeigneten Schutzgruppe, die Bedingungen zur Umsetzung (Lösungsmittel, Temperatur, Dauer, etc.), aber auch die Möglichkeiten eine Schutzgruppe wieder zu entfernen sind im Stand der Technik bekannt (z.B. Philip Kocienski, Protecting Groups, 3rd ed. 2004, THIEME, Stuttgart, ISBN: 3131370033). Bevorzugte Schutzgruppen sind ggf. substituiertes Benzyl, Diphenylmethyl, Trityl, Tosyl, Mesyl oder Triflat, besonders bevorzugt ist ggf. substituiertes Benzyl.

### EXPERIMENTELLER TEIL

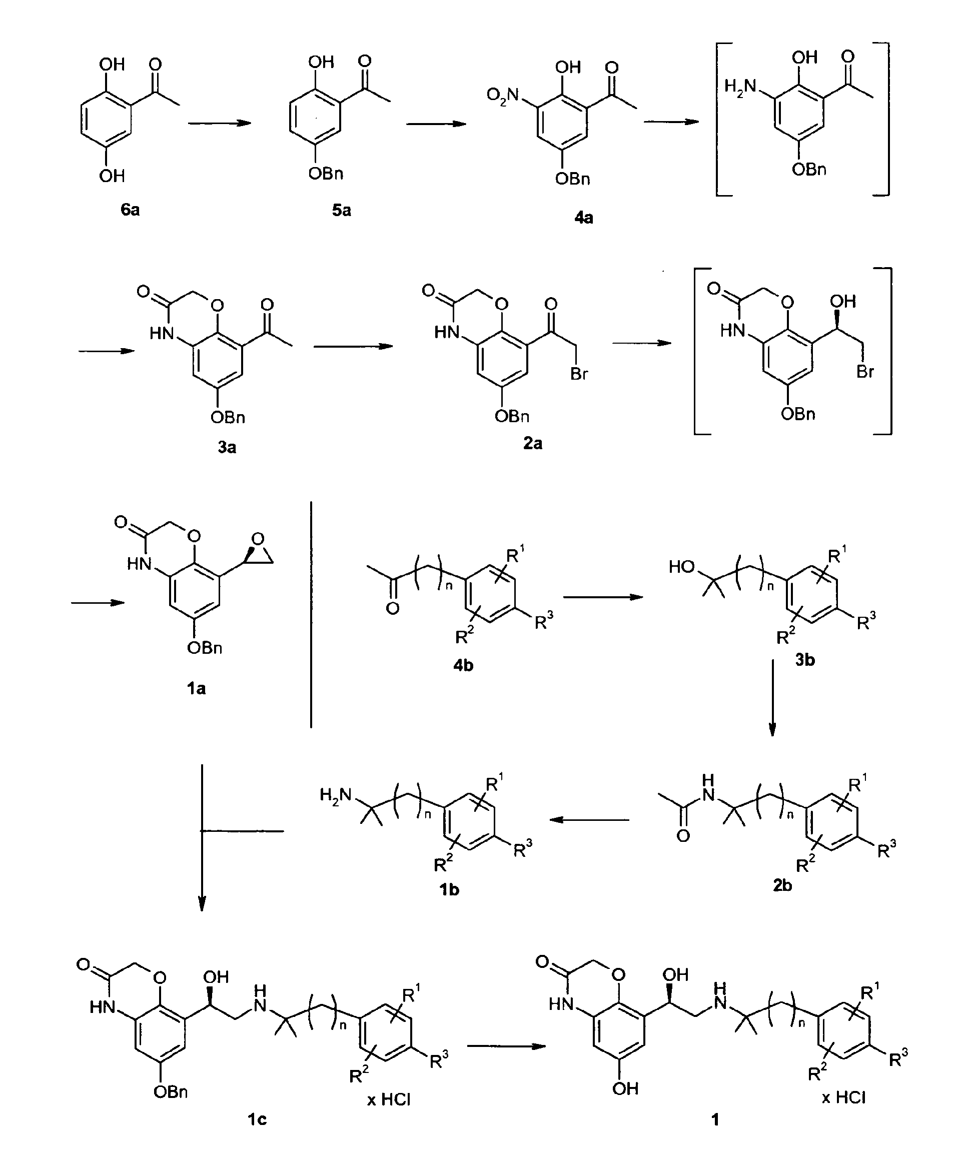

worin Bn die Bedeutung von Benzyl hat und
- n: 1 oder 2;
- R¹: Wasserstoff, Halogen, C₁₋₄-Alkyl oder O-C₁₋₄-Alkyl;
- R²: Wasserstoff, Halogen, C₁₋₄-Alkyl oder O-C₁₋₄-Alkyl;
- R³: Wasserstoff, C₁₋₄-Alkyl, OH, Halogen, O-C₁₋₄-Alkyl, O-C₁₋₄-Alkylen-COOH, O-C₁₋₄-Alkylen-COO-C₁₋₄-Alkyl;
bedeuten kann.

**8-[(1R)-1-Hydroxy-2-[[2-aryl-1,1-dimethyl-ethyl]-amino]ethyl]-6-(phenylmethoxy)-2H-1,4-benzoxazin-3(4H)-on-hydrochlorid** der Formel **1c**: 45 mol Aryl-1,1-dimethyl-ethylaminhydrochlorid **1b** werden in 12 L Wasser suspendiert und 60 L Toluol werden zugesetzt. 4,3 Kg Natronlauge (45%) werden unter Rühren zugegeben, und die Phasen werden getrennt. Zu der organischen Lösung werden 12 Kg (40 mol) 8-(2R)-Oxiranyl-6-(phenylmethoxy)-2H-1,4-benzoxazin-3(4H)-on **1a** zugegeben und auf Rückfluss aufgeheizt, (indem auch ca. 24 L Toluol abdestilliert werden) und bei dieser Temperatur 6 Stunden nachgerührt. Anschließend wird auf 55°C abgekühlt, 96 L Methylacetat zugegeben und bei dieser Temperatur innerhalb von 15 Minuten 3,8 kg (39 mol) Salzsäure (30%) zugegeben. Nach der Zugabe von ca. 5% von der Salzsäure wird mit Kristallen von **1c** angeimpft. Die entstandene Suspension wird auf 20°C abkühlt und 2 Stunden nachgerührt. Das Produkt wird zentrifugiert, mit 24 L Methylacetat nachgewaschen und im Vakuum bei 50°C getrocknet. Ausbeute (**1c**): 80-90%, Enantiomerenreinheit laut HPLC: 95,0 -99,5%.

**6-Hydroxy-8-[(1R)-1-hydroxy-2-[[2-aryl-1,1-dimethyl-ethyl]-amino]ethyl]-2H-1,4-benzoxazin-3(4H)-on-hydrochlorid** der Formel **1**: 19,49 mol 8-[(1R)-1-Hydroxy-2-[[2-(4-methoxyphenyl)-1,1-dimethyl-ethyl]-amino]ethyl]-6-(phenylmethoxy)-2H-1,4-benzoxazin-3(4H)-on-hydrochlorid der Formel **1c** werden im Hydrierreaktor vorgelegt und mit 50 L Methanol suspendiert. 125 g Palladium auf Kohle 10% (50% Wasser) werden in 20 L Methanol suspendiert und in den Hydrierreaktor eingezogen. Es wird bei 60°C Innentemperatur und bei 3 bar Wasserstoffdruck hydriert, bis keine Wasserstoffaufnahme mehr zu verzeichnen ist (ca. 1,5 Std). Der Katalysator wird abfiltriert und mit 20 L Methanol nachgespült. Es werden 165 L *i*-Propanol zudosiert, auf 50°C erhizt und unter schwachem Vakuum 180 L abdestilliert. Wenn keine Kristallbildung erfolgt, wird der Destillationsrückstand angeimpft. Anschließend wird innerhalb von 1 Stunde auf 0°C abgekühlt und danach bei 0°C 1 Stunde nachgerührt, abgesaugt und mit 30 L kaltem *i-*Propanol nachgewaschen und im Vakuum bei 45°C getrocknet.
Das Produkt (ca. 15,6 mol) wird in 26 Liter Methanol gelöst. Die entstandene Lösung wird auf 50°C geheizt, klarfiltriert, und das Druckfilter mit 6,6 Liter Methanol nachgespült. Es werden 53 L *i*-Propanol zudosiert, angeimpft und bei 50°C unter schwachem Vakuum ca. 50 L abdestilliert. Anschließend wird innerhalb von 1 Stunde auf 0°C abgekühlt und danach bei 0°C 1 Stunde nachgerührt, abgesaugt und mit 30 L kaltem *i*-Propanol nachgewaschen und im Vakuum bei 45°C getrocknet. Ausbeute (1): 65-80%.

**1-[2-Hydroxy-5-(phenylmethoxy)-phenyl]-ethanon:** 20 kg (131,4 mol) 2-Acetylhydrochinon **6a** werden in 150 L Methylisobutylketon gelöst und mit 19,98 kg (144,6 mol) Kaliumcarbonat versetzt. Bei 60°C werden 22,48 kg (131,5 mol) Benzylbromid zugegeben. Die Reaktionsmischung wird 20 Stunden bei 60°C gerührt. Die Reaktionsmischung wird auf 25°C gekühlt und der Feststoff wird abfiltriert. Das Filtrat wird zweimal mit je einer Lösung aus 0,96 kg (11,8 mol) Natriumhydroxid-Lsg. (50%) und 60 L Wasser bei 25°C gewaschen. Das Methylisobutylketon wird im Vakuum weitgehend abdestilliert, und der Rückstand wird in 80 L Methanol bei 60°C gelöst. Die Lösung wird auf 0°C abgekühlt und 1 Stunde bei dieser Temperatur zur Vervollständigung der Kristallisation gerührt. Ausbeute (**5a**): 24,07 kg (75,6%), Chemische Reinheit laut HPLC: 99,2%.

**1-[2-Hydroxy-3-nitro-5-(phenylmethoxy)-phenyl]-ethanon:** 10,00 kg (41,27 mol) 1-[2-Hydroxy-5-(phenylmethoxy)-phenyl]-ethanon **5a** werden in 50 L Essigsäure gelöst. Zu dieser Lösung werden bei 15 bis 20°C 4,40 kg (45,40 mol) Salpetersäure 65% zudosiert. Das Zulaufgefäß wird mit 4 L Essigsäure nachgespült. Das Reaktionsgemisch wird 1 Stunde nachgerührt. Nach Animpfen wird mit 50 L Wasser versetzt. Die erhaltene Suspension wird zur Vervollständigung der Kristallisation 1 Stunde bei 10°C gerührt. Das Produkt wird zentrifugiert und bei 50°C getrocknet. Ausbeute (**4a**): 10,34 kg (87,2%), Chemische Reinheit laut HPLC: 99,0%.

**8-Acetyl-6-(phenylmethoxy)-2*H*-1,4-benzoxazin-3(4*H*)-on**: 15,00 kg (52,22 mol) 1-[2-Hydroxy-3-nitro-5-(phenylmethoxy)-phenyl]-ethanon **4a**, 0,165 kg Platin(IV)oxid und 45 L 2-Methyltetrahydrofuran werden bei 3 bar Wasserstoffdruck und einer Innentemperatur von 25°C hydriert, bis keine Wasserstoffaufnahme mehr zu verzeichnen ist. Der Katalysator wird abfiltriert und mit 20 L 2-Methyltetrahydrofuran gewaschen. In einem weiteren Reaktor werden 23,09 kg (167,09 mol) Kaliumcarbonat vorgelegt, und das Reaktionsgemisch aus dem ersten Reaktor wird zugegeben. Es wird mit 22 L 2-Methyltetrahydrofuran nachgespült. Anschließend werden innerhalb von 30 Minuten 9,44 kg (83,55 mol) Chloracetylchlorid zur Suspension zudosiert. Nach 2,5 Stunden Reaktionszeit bei 65°C werden 101 L Wasser zugegeben. Die wässrige Phase wird bei 55°C abgetrennt. Anschließend werden aus der organischen Phase 34 L 2-Methyltetrahydrofuran im Vakuum abdestilliert. Nach Aufheizen auf Rückflusstemperatur werden innerhalb von 30 Minuten 180 L Methylcyclohexan unter Rückfluss zudosiert. Die erhaltene Suspension wird auf 20°C abgekühlt und zur Vervollständigung der Kristallisation noch 1 Stunde bei dieser Temperatur nachgerührt. Anschließend wird der Niederschlag abzentrifugiert, mit 113 L Methylcyclohexan gewaschen und bei 50°C getrocknet. Ausbeute (**3a**): 12,70 kg (81,8%), Chemische Reinheit laut HPLC: 98,4%.

**8-(Bromacetyl)-6-(phenylmethoxy)-2*H*-1,4-benzoxazin-3(4*H***)-**on**: 12,00 kg (40,36 mol) 8-Acetyl-6-(phenylmethoxy)-2*H*-1,4-benzoxazin-3(4*H*)-on **3a** werden in 108 L 1,4-Dioxan gelöst. Anschließend wird eine Lösung aus 24,33 kg (50,45 mol) Tetrabutylammoniumtribromid in 48 L 1,4-Dioxan und 12 L Methanol bei 20°C zur Suspension zudosiert. Der Reaktorinhalt wird 2 Stunden bei 20°C gerührt. Anschließend werden 72 L Wasser bei 20°C innerhalb von 15 Minuten zugegeben. Nach Abkühlung auf 3°C wird 1 Stunde gerührt, zentrifugiert und mit einer Mischung aus 9 L 1,4-Dioxan und 4,5 L Wasser gewaschen. Anschließend wird mit 60 L Wasser gewaschen und im Vakuum bei 50°C getrocknet. Ausbeute (**2a**): 11,29 kg (74,4%), Chemische Reinheit laut HPLC: 98,0%.

**8-(2R)-Oxiranyl-6-(phenylmethoxy)-2*H*-1,4-benzoxazin-3(4*H*)**-**on:** 20,00 kg (31,90 mol) 8-(Bromacetyl)-6-(phenylmethoxy)-2*H*-1,4-benzoxazin-3(4*H*)-on **2a** werden in 200 L Tetrahydrofuran gelöst und auf -30°C abgekühlt. Es werden 50,3 kg (70,18 mol) (-)-DIP-Chlorid in Heptan 65% innerhalb von 1 Stunden zudosiert. Das Reaktionsgemisch wird 2 Stunden nachgerührt und auf 0°C erwärmt. Bei dieser Temperatur werden 18,9 kg (143,54 mol) Natriumhydroxid-Lsg. (50%), mit 40 L Wasser gemischt, zudosiert. Anschließend wird das Zulaufgefäß mit 10 L Wasser gespült. Der pH-Wert am Ende der Zugabe sollte 13-13,5 betragen. Es wird auf 20°C erwärmt und 1 Stunde nachgerührt. Eine Mischung aus 7,8 Kg (80 mol) Salzsäure (37%) techn. und 80 L Wasser wird solange zudosiert, bis ein pH-Wert von 8,5 erreicht ist. Nach Zugabe von 100 L Ethylacetat wird auf 45°C erwärmt. Nach Phasentrennung wird aus der organischen Phase ein Teil des Lösungsmittels (ca. 280 L) abdestilliert, der Rückstand wird mit 160 L *tert*-Butyl-methylether versetzt, auf 0°C abgekühlt und 1 Stunde nachgerührt. Das Produkt wird isoliert, mit *tert*-Butylmethylether gewaschen und im Vakuum bei 50°C getrocknet. Ausbeute (**1a**): 13,96 kg (87,0%), Enantiomeren Reinheit laut HPLC: 98,3%.

**Verbindungen der Formel 3b:** 24,68 kg (72,6 mol) Methylmagnesiumchlorid (22%ige Lösung in THF) werden in 35 L Toluol gelöst und auf 16°C abgekühlt. Bei 16 - 22°C wird eine Lösung aus 60,9 mol Arylaceton der Formel **4b** und 10 L Toluol zudosiert und bei 22°C 1 Stunde gerührt. Die Reaktionslösung wird zu einer Mischung von 45 L Wasser und 5,22 kg (51,1 mol) Schwefelsäure bei einer Temperatur von 2-17°C zudosiert. Das zweiphasige Gemisch wird gerührt, und die wässrige Phase wird abgetrennt. Die organische Phase wird mit einer Lösung aus 1,00 kg (11,9 mol) Natriumhydrogencarbonat und 11 L Wasser gewaschen. Das Lösungsmittel wird im Vakuum vollständig abdestilliert. Der Rückstand wird in 65,5 L *n*-Heptan gelöst. Nach Abkühlung auf 2°C wird die Reaktionsmischung 3 Stunden bei dieser Temperatur gerührt. Anschließend wird das Produkt isoliert, mit 17,5 L *n*-Heptan gewaschen und im Vakuum bei 25°C getrocknet. Ausbeute (**3b**): 75-80%, Chemische Reinheit laut HPLC: 98.9-99,9%.

**Verbindungen der Formel 2b:** 55,48 mol 1-Aryl-2-methyl-propan-2-ol der Formel 3b werden in 6,83 kg (166,44 mol) Acetonitril und 13 L Essigsäure vorgelegt und auf 40°C erwärmt. 5,66 kg (55,48 mol) Schwefelsäure werden bei 50 - 55°C zudosiert. Anschließend wird das Gemisch 3 Stunden bei 50°C gerührt. In einem zweiten Reaktor werden 160 L Wasser, 20 L *tert*-Butylmethylether und 21 L Methylcyclohexan auf 10°C gekühlt. Der Inhalt des ersten Reaktors wird in den zweiten Reaktor überführt. Der pH Wert des Reaktorinhalts wird mit ca. 40 I Ammoniaklösung (25%) auf 9,5 eingestellt. Die Suspension wird auf 5°C abgekühlt und 1 Stunde bei dieser Temperatur gerührt. Das Produkt wird abzentrifugiert und mit 30 L Wasser sowie mit einem Gemisch aus 7,5 L ***tert-***Butylmethylether und 7,5 L Methylcyclohexan gewaschen. Das feuchte Produkt wird in 25 L Ethanol (96%ig) auf 75°C erwärmt und bei dieser Temperatur mit 30 L Wasser versetzt. Die Lösung wird 15 Minuten bei 85°C gerührt, dann auf 2°C abgekühlt und 1 Stunde bei dieser Temperatur gerührt. Das Produkt wird isoliert, mit einer Mischung aus 5 L Wasser und 5 L Ethanol (96%ig) gewaschen und getrocknet. Ausbeute (**2b**): 65-71%, Chemische Reinheit laut HPLC: 98.6-99,8%.

**Verbindungen der Formel 1b:** Eine Mischung aus 136 mol N-[2-Aryl-1,1-dimethyl-ethyl]-acetamid der Formel **2b,** 27,3 kg NaOH (678 mol), 30 L Ethoxyethanol und 15 L Ethylenglykol wird 3 Stunden auf 150°C erhitzt. Nach Abkühlung auf 50-80°C wird das Gemisch mit 90 L Wasser und 90 L Toluol verdünnt. Die Phasen werden getrennt und die organische Phase wird noch einmal mit 60 L Wasser gewaschen. Die organische Phase wird mit 13,4 kg Salzsäure (136 mol) versetzt. Nach der Destillation von 9,5 L Azeotrop wird die Lösung bei über 80°C angeimpft und über 1 Stunde auf Raumtemperatur abgekühlt. Das Produkt wird zentrifugiert, mit 60 Liter Toluol nachgewaschen und im Vakuum bei 50°C getrocknet. Ausbeute (**1b**): 85-95%, Chemische Reinheit laut HPLC: >99,5%.

In den oben genannten Synthesen für die Verbindungen der Formeln **3b, 2b** und **1b** können die Reste R¹, R² und R³ beispielsweise folgende Bedeutung haben:

| | **R¹** | **R²** | **R³** |
|---|---|---|---|
| Beispiel 1 | H | H | OMe |
| Beispiel 2 | 2-F | H | F |
| Beispiel 3 | 3-F | 5-F | H |
| Beispiel 4 | H | H | OEt |
| Beispiel 5 | H | H | F |

In Analogie zu dem vorstehend beschriebenen Herstellungsverfahren können somit die *R-*Formen der nachfolgenden Verbindungen der Formel 1 erhalten werden:
- 6-Hydroxy-8-{1-hydroxy-2-[2-(4-methoxy-phenyl)-1,1-dimethyl-ethylamino]-ethyl}-4H-benzo[1,4]oxazin-3-on;
- 8-{2-[2-(2,4-Difluor-phenyl)-1,1-dimethyl-ethylamino]-1-hydroxy-ethyl}-6-hydroxy-4H-benzo[1,4]oxazin-3-on;
- 8-{2-[2-(3,5-Difluor-phenyl)-1,1-dimethyl-ethylamino]-1-hydroxy-ethyl}-6-hydroxy-4H-benzo[1,4]oxazin-3-on;
- 8-{2-[2-(4-Ethoxy-phenyl)-1,1-dimethyl-ethylamino]-1-hydroxy-ethyl}-6-hydroxy-4H-benzo[1,4]oxazin-3-on;
- 8-{2-[2-(4-Fluor-phenyl)-1,1-dimethyl-ethylamino]-1-hydroxy-ethyl}-6-hydroxy-4H-benzo[1,4]oxazin-3-on.

## Patentansprüche

1. Verfahren zur Herstellung von Verbindungen der Formel 1, 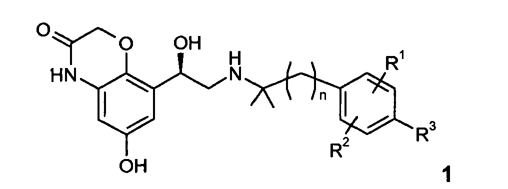 worin
n 1 oder 2;
R¹ Wasserstoff, Halogen, C₁₋₄-Alkyl oder O-C₁₋₄-Alkyl;
R² Wasserstoff, Halogen, C₁₋₄-Alkyl oder O-C₁₋₄-Alkyl;
R³ Wasserstoff, C₁₋₄-Alkyl, OH, Halogen, O-C₁₋₄-Alkyl, O-C₁₋₄-Alkylen-COOH oder O-C₁₋₄-Alkylen-COO-C₁₋₄-Alkyl;
bedeuten, **dadurch gekennzeichnet, dass** eine Verbindung der Formel **1a**, 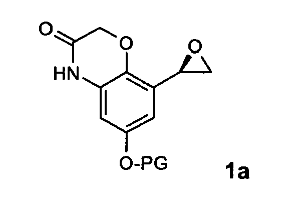 worin PG für eine Schutzgruppe steht, mit einer Verbindung der Formel **1b**, 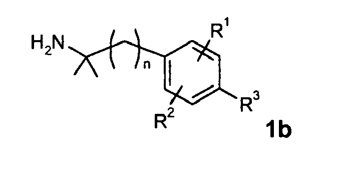 worin R¹, R², R³ und n die oben genannte Bedeutung haben, in Toluol in einem Zeitraum von 3-9 Stunden zu einer Verbindung der Formel **1c**, 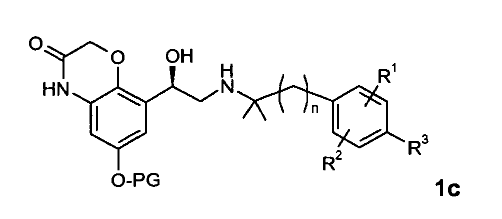 worin R¹, R², R³, n und PG die oben genannten Bedeutungen haben, umgesetzt und aus dieser durch Abspaltung der Schutzgruppe PG die Verbindung der Formel 1 erhalten wird.

2. Verfahren zur Herstellung von Verbindungen der Formel **1** nach Anspruch 1, worin
n 1 oder 2;
R¹ Wasserstoff, Halogen oder C₁₋₄-Alkyl;
R² Wasserstoff, Halogen oder C₁₋₄-Alkyl;
R³ Wasserstoff, C₁₋₄-Alkyl, OH, Halogen, O-C₁₋₄-Alkyl, O-C₁₋₄-Alkylen-COOH oder O-C₁₋₄-Alkylen-COO-C₁₋₄-Alkyl;
bedeuten.

3. Verfahren zur Herstellung von Verbindungen der Formel **1** nach Anspruch 1, worin
n 1 oder 2;
R¹ Wasserstoff, Fluor, Chlor, Methyl oder Ethyl;
R² Wasserstoff, Fluor, Chlor, Methyl oder Ethyl;
R³ Wasserstoff, C₁₋₄-Alkyl, OH, Fluor, Chlor, Brom, O-C₁-₄-Alkyl, O-C₁₋₄-Alkylen-COOH oder O-C₁₋₄-Alkylen-COO-C₁₋₄-Alkyl;
bedeuten.

4. Verfahren zur Herstellung von Verbindungen der Formel **1** nach Anspruch 1, worin
n 1 oder 2;
R¹ Wasserstoff, Methyl oder Ethyl;
R² Wasserstoff, Methyl oder Ethyl;
R³ Wasserstoff, Methyl, Ethyl, OH, Methoxy, Ethoxy, O-CH₂-COOH, O-CH₂-COO-Methyl oder O-CH₂-COO-Ethyl;
bedeuten.

5. Verfahren zur Herstellung von Verbindungen der Formel **1** nach Anspruch 1, worin
n 1 oder 2;
R¹ Wasserstoff oder Methyl;
R² Wasserstoff oder Methyl;
R³ Wasserstoff, Methyl, OH, Methoxy, O-CH₂-COOH oder O-CH₂-COO-Ethyl;
bedeuten.

6. Verfahren nach einem der Ansprüche 1 bis 5, worin die Verbindung der Formel **1a** durch Umsetzung einer Verbindung der Formel **2a**, 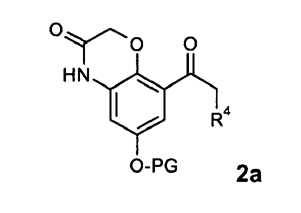 worin PG die in Anspruch 1 genannte Bedeutung hat und R⁴ Halogen bedeutet, in Gegenwart einer Diisopinocampheylchloroboran/Heptan-Lösung hergestellt wird.

## Claims

1. Process for preparing compounds of formula 1, 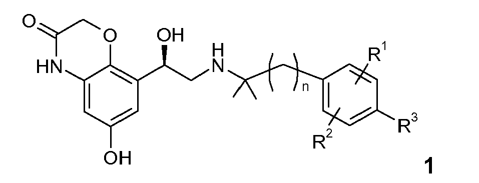 wherein
n denotes 1 or 2;
R¹ denotes hydrogen, halogen, C₁₋₄-alkyl or O-C₁₋₄-alkyl;
R² denotes hydrogen, halogen, C₁₋₄-alkyl or O-C₁₋₄-alkyl;
R³ denotes hydrogen, C₁₋₄-alkyl, OH, halogen, O-C₁₋₄-alkyl, O-C₁₋₄-alkylene- COOH, O-C₁₋₄-alkylene-COO-C₁₋₄-alkyl;
**characterised in that** a compound of formula **1a**, 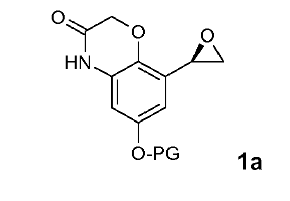 wherein PG denotes a protective group, is reacted with a compound of formula **1b**, 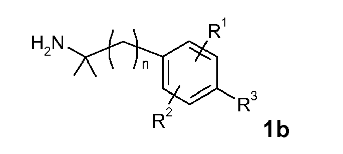 wherein R¹, R², R³ and n have the meanings given above, in toluene, over a period of 3-9 hours, to form a compound of formula **1c**, 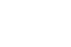 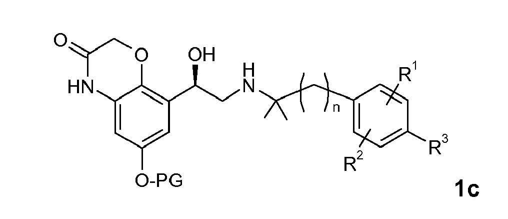 wherein R¹, R², R³, n and PG have the meanings given above, and the compound of formula **1** is obtained therefrom by cleaving the protective group PG.

2. Process for preparing compounds of formula **1** according to claim 1, wherein
n denotes 1 or 2;
R¹ denotes hydrogen, halogen or C₁₋₄-alkyl;
R² denotes hydrogen, halogen or C₁₋₄-alkyl;
R³ denotes hydrogen, C₁₋₄-alkyl, OH, halogen, O-C₁₋₄-alkyl, O-C₁₋₄-alkylene-COOH or O-C₁₋₄-alkylene-COO-C₁₋₄-alkyl.

3. Process for preparing compounds of formula **1** according to claim 1, wherein
n denotes 1 or 2;
R¹ denotes hydrogen, fluorine, chlorine, methyl or ethyl;
R² denotes hydrogen, fluorine, chlorine, methyl or ethyl;
R³ denotes hydrogen, C₁₋₄-alkyl, OH, fluorine, chlorine, bromine, O-C₁₋₄-alkyl, O-C₁₋₄-alkylene-COOH or O-C₁₋₄-alkylene-COO-C₁₋₄-alkyl.

4. Process for preparing compounds of formula **1** according to claim 1, wherein
n denotes 1 or 2;
R¹ denotes hydrogen, methyl or ethyl;
R² denotes hydrogen, methyl or ethyl;
R³ denotes hydrogen, methyl, ethyl, OH, methoxy, ethoxy, O-CH₂-COOH, O-CH₂-COO-methyl or O-CH₂-COO-ethyl.

5. Process for preparing compounds of formula **1** according to claim 1, wherein
n denotes 1 or 2;
R¹ denotes hydrogen or methyl;
R² denotes hydrogen or methyl;
R³ denotes hydrogen, methyl, OH, methoxy, O-CH₂-COOH or O-CH₂-COO-ethyl.

6. Process according to one of claims 1 to 5, wherein the compound of formula **1a** is prepared by reacting a compound of formula **2a**, 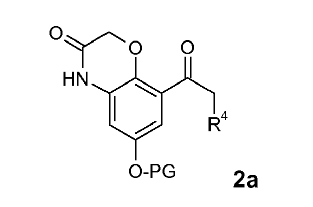 wherein PG has the meaning given in claim 1 and R⁴ denotes halogen, in the presence of a diisopinocampheylchloroborane/heptane solution.

## Revendications

1. Procédé de production de composés de formule 1, 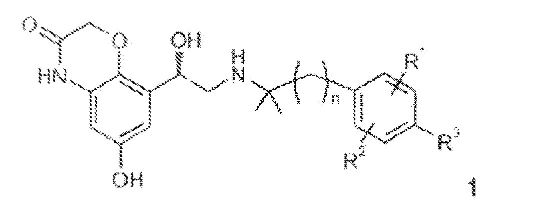 dans laquelle
n vaut 1 ou 2 ;
R¹ est hydrogène, halogène, alkyle en C₁₋₄ ou O-alkyle en C₁₋₄ ;
R² est hydrogène, halogène, alkyle en C₁₋₄ ou O-alkyle en C₁₋₄ ;
R³ est hydrogène, alkyle en C₁₋₄, OH, halogène, O-allyle en C₁₋₄, O-alkylène en C₁₋₄-COOH ou O-alkylène en C₁₋₄-COO-alkyle en C₁₋₄ ;
**caractérisé en ce que** l'on fait réagir un composé de formule 1a, 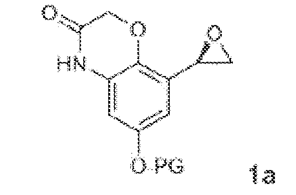 dans laquelle PG représente un groupe protecteur, avec un composé de formule 1b, 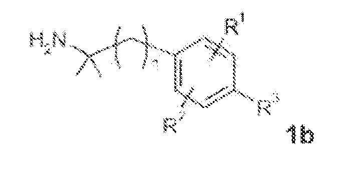 dans laquelle R¹, R², R³ et n ont la signification indiquée ci-dessus, dans du toluène pendant une durée de 3 à 9 heures pour donner un composé de formule 1c, 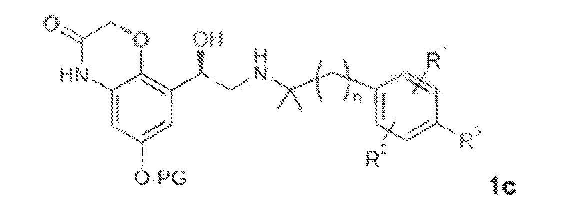 dans laquelle R¹, R², R³, n et PG ont les significations indiquées ci-dessus, et, par dissociation du groupe protecteur PG, on obtient à partir dudit composé 1c le composé de formule 1.

2. Procédé de production de composés de formule 1 selon la revendication 1, formule dans laquelle n vaut 1 ou 2 ;
R¹ est hydrogène, halogène ou alkyle en C₁₋₄ ;
R² est hydrogène, halogène ou alkyle en C₁₋₄ ;
R³ est hydrogène, alkyle en C₁₋₄, OH, halogène, O-allyle en C₁₋₄, O-alkylène en C₁₋₄-COOH ou O-alkylène en C₁₋₄-COO-alkye en C₁₋₄.

3. Procédé de production de composés de formule 1 selon la revendication 1, formule dans laquelle n vaut 1 ou 2 ;
R¹ est hydrogène, fluor, chlore, méthyle ou éthyle ;
R² est hydrogène, fluor, chlore, méthyle ou éthyle ; R³ est hydrogène, alkyle en C₁₋₄, OH, fluor, chlore, brome, O-alkyyle en C₁₋₄, O-alkylène en C₁₋₄-COOH ou O-alkylène en C₁₋₄-COO-alkyle en C₁₋₄.

4. Procédé de production de composés de formule 1 selon la revendication 1, formule dans laquelle n vaut 1 ou 2 ;
R¹ est hydrogène, méthyle ou éthyle ;
R² est hydrogène, méthyle ou éthyle ;
R³ est hydrogène, méthyle, éthyle, OH, méthoxy, éthoxy, O-CH₂-COOH, O-CH₂-COO-méthyle ou O-CH₂-COO-éthyle.

5. Procédé de production de composés de formule 1 selon la revendication 1, formule dans laquelle n vaut 1 ou 2 ;
R¹ est hydrogène ou méthyle ;
R² est hydrogène ou méthyle ;
R³ hydrogène, méthyle, OH, méthoxy, O-CH₂-COOH ou O-CH₂-COO-éthyle,

6. Procédé selon l'une des revendications 1 à 5, dans lequel le composé de formule la est obtenu en faisant réagir un composé de formule 2a, 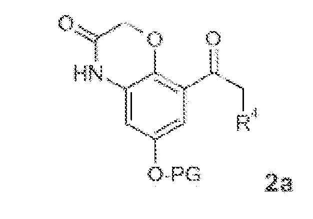 dans laquelle PG a la signification indiquée dans la revendication 1 et R⁴ est un halogène, en présence d'une solution de diisopinocamphéylchloroborane dans de l'heptane.
